# EUROPEAN PATENT SPECIFICATION

(11) **EP 3 201 312 B1**
(45) Date of publication and mention of the grant of the patent: **04.03.2020**
(21) Application number: 15847901.4
(22) Date of filing: 22.09.2015
(51) Int. Cl.: C12M 1/34, G01N 33/566

(54) **METHODS AND DEVICES RELATING TO THE DETECTION OF ORAL CANCER BIOMARKERS**
VERFAHREN UND VORRICHTUNGEN IM ZUSAMMENHANG MIT DER DETEKTION VON MUNDKREBSMARKERN
PROCÉDÉS ET DISPOSITIFS RELATIFS À LA DÉTECTION DE BIOMARQUEURS DU CANCER BUCCAL

(30) Priority: 30.09.2014 GB 201417281
(43) Date of publication of application: 09.08.2017
(73) Proprietor: GE Healthcare UK Limited, Little Chalfont, Buckinghamshire HP7 9NA (GB)
(72) Inventor: HORTON,Jeffrey Kenneth, Cardiff South Wales CF14 7YT (GB); TATNELL, Peter James, Cardiff South Wales CF14 7YT (GB)
(74) Representative: Aldenbäck, Ulla Christina
(86) International application number: PCT/US2015/051498
(87) International publication number: WO 2016/053689

(56) References cited:
- US-A1- 2007 249 961
- US-A1- 2011 070 604
- US-A1- 2011 318 763
- JOEL A KOOREN ET AL: "Evaluating the potential of a novel oral lesion exudate collection method coupled with mass spectrometry-based proteomics for oral cancer biomarker discovery", CLINICAL PROTEOMICS, BIOMED CENTRAL LTD, LONDON, UK, vol. 8, no. 1, 13 September 2011 (2011-09-13), page 13, XP021109202, ISSN: 1559-0275, DOI: 10.1186/1559-0275-8-13
- SPAFFORD M F ET AL: "Detection of head and neck squamous cell carcinoma among exfoliated oral mucosal cells by microsatellite analysis", CLINICAL CANCER RESEARCH, THE AMERICAN ASSOCIATION FOR CANCER RESEARCH, US, vol. 7, no. 3, 1 March 2001 (2001-03-01), pages 607-612, XP002376394, ISSN: 1078-0432
- S. HU ET AL: "Salivary Proteomics for Oral Cancer Biomarker Discovery", CLINICAL CANCER RESEARCH, vol. 14, no. 19, 1 October 2008 (2008-10-01), pages 6246-6252, XP55456516, US ISSN: 1078-0432, DOI: 10.1158/1078-0432.CCR-07-5037
- LI Y ET AL: "Salivary transcriptome diagnostics for oral cancer detection", CLINICAL CANCER RESEARCH, THE AMERICAN ASSOCIATION FOR CANCER RESEARCH, US, vol. 10, no. 24, 15 December 2004 (2004-12-15), pages 8442-8450, XP002448846, ISSN: 1078-0432, DOI: 10.1158/1078-0432.CCR-04-1167
- D. ELASHOFF ET AL: "Prevalidation of Salivary Biomarkers for Oral Cancer Detection", CANCER EPIDEMIOLOGY, BIOMARKERS AND PREVENTION., vol. 21, no. 4, 1 February 2012 (2012-02-01), pages 664-672, XP055381398, US ISSN: 1055-9965, DOI: 10.1158/1055-9965.EPI-11-1093
- GIESE M ET AL: "pSJ-Mutationsnachvveis in Abstrichen der Mundschieimhaut von Patienten mit oralem Plattenepithelkarzinom - [Detection of p53 mutation in mouth mucosa smears of patients with oral squamous epithelial carcinoma]", MUND-, KIEFER UND GESICHTSCHIRURGIE, SPRINGER, BERLIN, DE, vol. 5, no. 1, 1 January 2001 (2001-01-01) , pages 37-43, XP009503864, ISSN: 1432-9417, DOI: 10.1007/S100060000233 [retrieved on 2013-01-29]
- LIAO P-H ET AL: "Mutation of p53 gene codon 63 in saliva as a molecular marker for oral squamous cell carcinomas", ORAL ONCO, ELSEVIER SCIENCE, OXFORD, GB, vol. 36, no. 3, 1 May 2000 (2000-05-01), pages 272-276, XP004286690, ISSN: 1368-8375, DOI: 10.1016/S1368-8375(00)00005-1
- THOMAS SHPITZER ET AL: "A comprehensive salivary analysis for oral cancer diagnosis", JOURNAL OF CANCER RESEARCH AND CLINICAL ONCOLOGY, SPRINGER, BERLIN, DE, vol. 133, no. 9, 4 May 2007 (2007-05-04), pages 613-617, XP019542570, ISSN: 1432-1335, DOI: 10.1007/S00432-007-0207-Z
- M DHIVYALAKSHMI ET AL: "Expression of salivary biomarkers - Alkaline phosphatase & lactate dehydrogenase in oral leukoplakia", INTERNATIONAL JOURNAL OF CHEMTECH RESEARCH, vol. 6, no. 5, 1 January 2014 (2014-01-01) , pages 3014-3018, XP55472059, United States ISSN: 3014-3018
- PRIYASHIRISH JOSHI ET AL: "A study of salivary lactate dehydrogenase isoenzyme levels in patients with oral leukoplakia and squamous cell carcinoma by gel electrophoresis method", JOURNAL OF ORAL AND MAXILLOFACIAL PATHOLOGY : JOMFP, vol. 18, no. 4, 1 January 2014 (2014-01-01), page 39, XP55472063, IN ISSN: 0973-029X, DOI: 10.4103/0973-029X.141342
- NAGLER, R ET AL.: 'Concomitant Analysis Of Salivary Tumor Markers-A New Diagnostic Tool For Oral Cancer.' CLIN CANCER RES. vol. 12, no. 13, 01 July 2006, pages 3979 - 3984, XP002451711 DOI: 10.1158/1078-0432.CCR-05-2412

### SEQUENCE LISTING

<110> GE HEALTHCARE UK LIMITED
   Horton, Jeffrey
   Tatnell, Peter
<120> Methods and devices relating to the detection of oral cancer biomarkers
<130> 276959-2
<150> GB 1417281.1
   <151> 2014-09-30
<160> 10
<170> PatentIn version 3.5
<210> 1
   <211> 30
   <212> DNA
   <213> Artificial Sequence
<220>
   <223> Synthetic Oligonucleotide
<400> 1
   gagaatcgct tgaacccggg aggtgtaggt 30
<210> 2
   <211> 30
   <212> DNA
   <213> Artificial Sequence
<220>
   <223> Synthetic Oligonucleotide
<400> 2
   cctgctgatg cctggcctct ttcttctttg 30
<210> 3
   <211> 30
   <212> DNA
   <213> Artificial Sequence
<220>
   <223> Synthetic Oligonucleotide
<400> 3
   catcagcccg tctaggaacc cagtcatcag 30
<210> 4
   <211> 30
   <212> DNA
   <213> Artificial Sequence
<220>
   <223> Synthetic Oligonucleotide
<400> 4
   ctccttcatc ccgctacacc acgcatacac 30
<210> 5
   <211> 22
   <212> DNA
   <213> Artificial Sequence
<220>
   <223> Synthetic Oligonucleotide
<400> 5
   gcactggctt aggagttgga ct 22
<210> 6
   <211> 21
   <212> DNA
   <213> Artificial Sequence
<220>
   <223> Synthetic Oligonucleotide
<400> 6
   acagacaccc aggcctactt g 21
<210> 7
   <211> 29
   <212> DNA
   <213> Artificial Sequence
<220>
   <223> Synthetic Oligonucleotide
<400> 7
   gcactggctt aggagttgga cttcaaacc 29
<210> 8
   <211> 25
   <212> DNA
   <213> Artificial Sequence
<220>
   <223> Synthetic Oligonucleotide
<400> 8
   caactgctga aagagatgcg gtggg 25
<210> 9
   <211> 24
   <212> DNA
   <213> Artificial Sequence
<220>
   <223> Synthetic Oligonucleotide
<400> 9
   agcccttggg gasttgaatt gctg 24
<210> 10
   <211> 27
   <212> DNA
   <213> Artificial Sequence
<220>
   <223> Synthetic Oligonucleotide
<400> 10
   gcactccaga ggacagcrgt gtcaata 27

## Description

### FIELD OF THE INVENTION

The present invention relates to an ex vivo method for detecting and quantifying plural oral biomarkers within biological material present in a human oral cavity when oral cancerous activity is present.

### BACKGROUND OF THE INVENTION

Oral cancer has emerged as an alarming public health problem with increasing incidence and mortality rates all over the world. Oral cancer (predominantly oral squamous cell carcinoma, OSCC) is the sixth most common human malignancy, with a high rate of morbidity and a 5-year mortality rate of approximately 50% which has not changed significantly in more than 50 years. Therefore, the implementation of newer screening and early detection approaches are of utmost importance which could reduce the morbidity and mortality associated with this disease. The therapeutic modality currently offered to OSCC patients is based on tumour metastasis nodes criteria and on histological grading. Unfortunately, these predictors are subjective and relatively unreliable, as two tumours with identical staging and grading often behave very differently; though one responds to therapy, the other may be lethal. Thus, there has been an ever-growing effort dedicated to the basic research of oral cancer, focusing on the identification of biological indicators for the diagnosis of its biological nature and aggressiveness.

Biomarkers have been suggested previously to be related to OSCC (mostly by tissue analysis) for example: carbonyls, 8-oxoguanine DNA glycosylase (OGG1), mammary serine protease inhibitor (Maspin), Ki67, phosphorylated-Src (phospho-Src), Cyclin D1 (CycD1), metalloproteinase-9 (MMP-9) and lactate dehydrogenase (LDH). An additional target might involve HPV. A review of one set of biomarkers has been described in Cheng et al. Clincal Translational Medicine 2014 3:3 .

Plural biomarkers have not been used clinically simultaneously.

Oral cavity sampling, a non-invasive alternative to serum testing, has been identified by the inventors of the present invention as an effective modality for diagnosis and prognosis predicting of oral cancer as well as for monitoring the patient's post-therapy status. However, no oral sample device or test is currently offered commercially to detect, or predict the progression of OSCC.

Proposed herein, is the collection/storage of oral cavity biological material on solid supports consisting of base papers such as Whatman 903 ® cellulose filter paper, which will facilitate oral cancer screening approaches. Alternative sample collection materials could include other solid materials such as alginates and even material coated with non-hazardous chemicals. It is important that a solid support material is used that does not cause the denaturation of the protein as denaturation may restrict immunological detection systems in approaches like ELISA, Western blot etc.

The incorporation of base paper, alginate or similar non-hazardous and non-denaturing solid supports into the oral collection and storage device will permit the following techniques; i) direct sampling of cells from the oral cavity without any significant permanent harm to the patient and ii) during the molecular detection phase, non-coated (and certain chemically-coated) solid supports can be used in direct/punch-in workflows in which a small portion of the collected sample is excised and placed directly into a solution of the detection reagent. These techniques facilitate the direct sampling of saliva potentially containing oral cancer biomarkers.

### SUMMARY OF THE INVENTION

The inventors have established that the proximity of saliva and oral cancer lesions makes the measurement of tumour markers in the oral cavity an attractive alternative to serum and tissue testing. Further the inventors have established that any DNA, RNA, protein molecules, and any other analytes of interest, derived from the living cancer cells can be conveniently obtained from saliva and potentially collected and stored on a novel swab device. Novel testing described herein involves a punch-in system with direct analysis of the biomarker obtained on the swab device (by means of direct PCR, RT PCR, protein, enzyme analysis, capillary electrophoresis, TOF MS, and other known methods).

The invention provides a method according to claim 1 having preferred features defined by claims dependent on claim 1.

### BRIEF DESCRIPTION OF THE DRAWINGS

The invention can be put into effect in numerous ways, illustrative embodiments of which are described below with reference to the drawings, wherein:
Figure 1 shows a chart of protein recovery results;
Figures 2a, 2b, 2c and 2d show charts of enzyme recovery results;
Figures 3a and 3b show charts of further protein recovery results;
Figures 4a, 4b, 4c, and 5a show DNA recovery results;
Figures 6a and 6b show RNA recovery results;
Figures 7a to 11b show a swab device suitable for the sampling techniques described herein; and
Figures 12a and 12b show a holder for the swab device shown in the previous Figures

### DETAILED DESCRIPTION OF THE INVENTION

The invention, together with its objects and the advantages thereof, may be understood better by reference to the following description taken in conjunction with the accompanying drawings, in which like reference numerals identify like elements in the Figures.

Detection of specific biomarkers for oral cancer is the most likely effective method for screening, diagnosis, staging and follow-up for this malignancy. Unlike other deep tissue cancers, oral cancer is located only in the oral cavity, which term is herein intended to include mouth, and upper throat (pharynx) in humans. Hence, the direct contact between saliva and cells from the oral cancer lesion makes the measurement of tumour markers in saliva and lesion areas an attractive alternative to invasive serum and tissue testing. The DNA, RNA, protein molecules and other analytes of interest derived from the living cancer cells can be conveniently collected from saliva and lesions on a swab-like device, then stored if required. Herein the term 'biomarker(s)' includes that collected DNA, RNA, protein molecules and other analytes of interest, as well as other secondary biological material which results from, or forms as a consequence, of the presence of those biomarkers. The term 'biomarkers' also includes OSCC precursors such as viruses which may initiate cancerous activity. Herein 'cancerous activity', includes precancerous activity e.g. viral activity, associated with OSCC.

Thus, herein is described a novel method suitable for collecting the oral cancer biomarker p53 gene codon 63, as a non-invasive alternative to serum, tissue based and biopsy biomarker collection. The method described is an effective method for the early diagnosis, prognosis and monitoring post therapy status.

Various technologies provide opportunities for high-throughput approaches to genomics and proteomics; which can be used to evaluate altered expressions of gene and protein targets in saliva of oral cancer patients. The collection/storage of oral biomarkers for example in saliva, and/or cancerous cells on solid supports can be followed by a simple direct or punch-in technique in which a sample on a solid support is added directly to detection reagents and subjected to biomarker detection methods such as, but not limited to, immunological assays and nucleic acid amplification technologies without the prior purification of the biomarkers or analytes of interest.

An alternative approach to the sampling device describe here is the use of the Whatman Easicollect ® device in combination with a non-chemically coated filter paper such as 903®. It is important that a solid support material is used that does not cause the denaturation of the protein as denaturation may restrict immunological detection systems to approaches like Western blot etc. The use of non-chemically-coated solid supports reduces the potential for harm to patients.

The incorporation of a uncoated plain filter paper, alginate or similar non-hazardous and non-denaturing solid supports into a mildly abrasive absorbent matrix into the saliva/cellular collection and storage device will permit direct sampling of the biomarkers, associated with potentially cancerous cells.

The inventors have reviewed the importance of several genomic and proteomic biomarkers and/or other analytes of interest for oral cancer and suggest the collection of these markers on solid supports supplied by Whatman mentioned above. The detection methods contemplated include gene expression or protein expression profiling using RT-PCR and/or the use of untreated paper and downstream protein based assays, as well as polymerase chain reaction (PCR), quantitative PCR (qPCR), microarray assays preceding qPCR, enzyme linked immunosorbent assay (ELISA), other immunological techniques, gel electrophoresis (2DE), capillary electrophoresis (TOF MS), high performance liquid chromatography (HPLC), mass spectrometry (MS), flame photometry, atomic absorption, and spectrophotometry.

**Table I - OSCC candidates:**

| Category | Potential OSCC biomarkers |
|---|---|
| DNA | P53 gene codon 63 |
| | D3S1234, D9S156, and D17S799 |
| | Mitochondrial DNAs (cytochrome c oxidase I and cytochrome c oxidase II) |
| | Hypermethylation of promoters in tumor suppressor genes: |
| | DAPK, DCC, MINT-31, TIMP-31, TIMP-3, p16, MGMT, CCNA1 |
| | MK167 gene |
| | Serpin B5 |
| | OGG1 gene |
| | TFRC gene |
| | HSPB1 gene |
| mRNA | IL-8 |
| | IL-1β |
| | DUSP1 (dual specificity phosphatase 1) |
| | H3F3A (H3 histone family 3A) |
| | OAZ1 (ornithin decarboxylase antizyme 1) |
| | S100P (S100 calcium binding protein P) |
| | SAT (spermidine/spermine N1-acetyltransferase EST) |
| Proteins and enzymes | Proliferation biomarker Ki67 |
| | Lactate Dehygrogenase LDH |
| | Matrix Metalloproteinase 1, 2 & 9 |
| | 8-Oxoguanine DNA Glcosylase OGG1 |
| | Serpin Peptidase inhibitor |
| | Tumour protein p53 auto-antibodies |
| | Mac-2 binding protein M2BP |
| | Transferrin Receptor C TfR1/TFRC |
| | HSP27 & 60 |
| | alpha B-crystalline |
| | ATP Synthase |
| | Beta Calgranulin |
| | Beta Myosin |
| | Tropomyosin |
| | Galectin-1 |
| | Maspin |
| | α-amylase |
| | IL-8 |
| | TNF-α |
| | IL-1 |
| | IL-6 |
| | Basic fibroblast growth factor |
| | Statherin |
| | Cyfra 21.1 |
| | Tissue polypeptide antigen (TPA) |
| | Cancer antigen 125 (CA125) |
| | Endothelin-1 |
| | IL-1β |
| | CD44 |
| | Total salivary protein |
| | Insulin growth factor 1 (IGF-1) |
| | CD59 |
| | Catalase |
| | Profilin |
| | S100A9/MRP14 |
| | M2BP |
| | Carcinoembryonic antigen (CEA) |
| | Carcinoma associated antigen CA-50 |
| | Salivary carbonyls |
| | Cyclin D1 |
| | Phosphorylated-Src |
| | Transferrin |
| | Zinc finger protein 501 peptide |
| | Hemopexin |
| | Haptoglobin |
| | Complement C3 |
| | Transthyretin |
| | α 1-antitrypsin |
| | α-L-fucosidase |
| | Telomerase |
| | Peroxidase |
| | Glutathione S-transferase (GST) |
| | Superoxide dismutase (SOD) |
| Non-organic ions | Na, Ca, F, and Mg |
| Oxidative stress-related molecules | Reactive nitrogen species (RNS) such as nitric oxide (NO), nitrites (NO2) and nitrates (NO3) |
| | 8-hydroxy-2-deoxyguanosine (8-OHdG) |
| | Glutathione |
| | Malondialdehyde (MDA) |
| Hormones | Cortisol |
| Metabolomics | Cadaverine, alpha-aminobutyric acid, |
| | C5H14N5, piperidine, taurine piperideine, |
| | pipercolic acid, C4H9N, C8H9N, pyrroline |
| | hydroxycarboxylic acid, betaine, C6H6N2O2, |
| | choline, |
| | carnitine, C4H5N2011 P |
| | Lactic acid |
| | Sialic acid |
| Amino acids | Alanine |
| | Phenylalanine |
| | Valine |
| | Leucine |
| | Isoleucine |
| | Tyrosine |
| | Histidine, |
| | Tryptophan |
| | Glutamic acid, |
| | Threonine |
| | Serine |
| | Glutamine |
| Other indicators | Human Papilloma Virus HPV |

Table 1 gives examples of biomarker candidates which are indicative of cancerous or precancerous activity in the oral cavity. Below are described various assays which can be conducted to detect the existence of these biomarkers in biological material collected on solid supports, and in some cases, to quantify them. While the list contains various proteins and enzymes, it is possible to detect these by means of mRNA analyses, rather than detect the protein itself and *vice versa.* Further below there is shown a swab device including a solid support suitable for the collection of the biological materials.

### Example 1: Direct measurement of interleukin (IL) from a solid support

Recombinant IL-2 ± carrier (R & D Systems; Cat. 202-IL-CF-10µg; lot AE4309112 and Cat. 202-IL-10µg; lot AE4309081 respectively) was dissolved in blood (TCS Biosciences) at 50 pg or 100 pg/µl. Aliquots (1 µl containing, 50 (B) or 100 (A) pg of IL-2) were applied to Whatman 903 filter papers.

These samples were allowed to dry overnight at ambient temperature and humidity. 3mm diameter punched disks were extracted from each paper type using the appropriately sized punch. Single discs were directly analysed for IL-2 with reagents from a fully 10 configured IL-2 Quantikine ELISA kit (R & D Systems, Cat. D2050, lot 273275). Direct assays were carried out "punch in well", i.e. where a portion of the 903 filter paper was punched out and deposited in a reaction well of a convention multiwell plate.

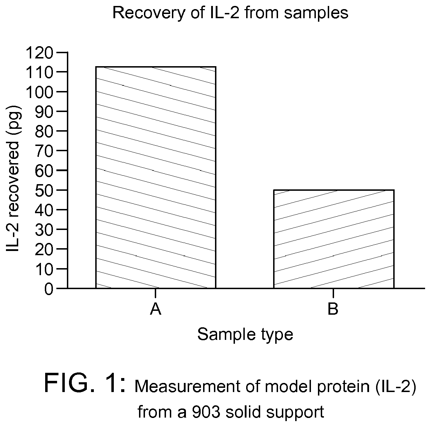

On completion of the assay the optical density was monitored at 450 nm. The recovery of IL-2 was determined by comparing values to a standard curve of known IL-2 concentrations. Recovery rates are shown in in Figure 1, and demonstrate that effective amounts of IL can be recovered then the IL is deposited on a solid support.

### Example 2: Model enzyme detection from cells or enzymes transferred to solid supports

Protein and enzyme testing was carried out with fully configured DNase and RNase Contamination Kits (DNase & RNase Alert QC Systems, catalogue codes AM1970 & AM1966, Life Technologies) according to the manufacturer's instructions.

### Dideoxyribonuclease (DNase)

In a first series of experiments, 0.125-0.5 U of DNase was applied to Whatman FTA and 903 papers in 10µl volumes. DNAse and RNase activity was measured as outlined below.

In a second series of experiments, 1.2mm punches were taken from 106 human embryonic stem cells (GE Healthcare; cell line ref: WCB307 GEHC 28) which had been applied to FTA and 903 papers in 10 µl volumes as above. DNAse and RNase activity was measured as outlined below.

In a third series of experiments, 1.2mm punches were taken from 106 human embryonic stem cells (GE Healthcare; cell line ref: WCB307 GEHC 28) containing either 0.5 U of DNase or 10 µU of RNase added to these cells which had been applied to FTA and 903 papers in 10 µl volumes.

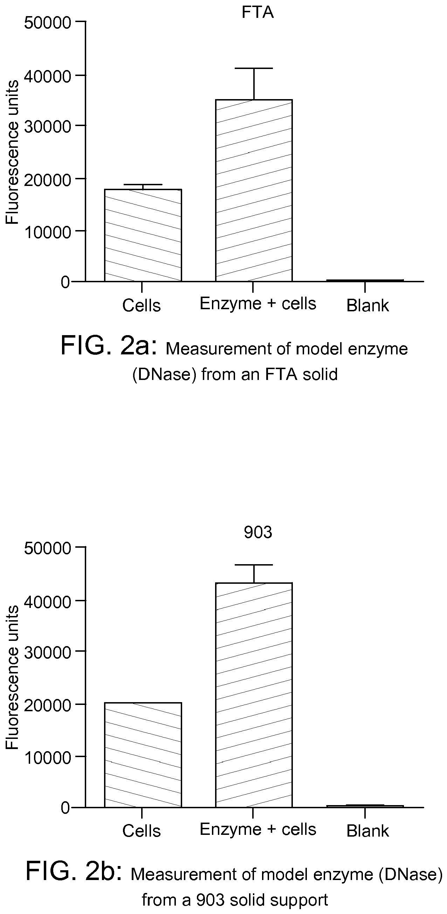

Detection of DNase activity was carried out as follows using a cleavable fluorescent-labelled DNase substrate. Each punch was ejected into separate wells of 96-well plates. Lyophilized DNase Alert Substrate was dissolved in TE buffer (1 ml) and dispensed (10 µl) into the test wells of the 96-well plate. 10X DNase Alert Buffer (10 µl) and nuclease-free water (80 µl) was added and the test solution (100 µl) incubated for 60 minutes at 37°C. The DNase Alert QC System Substrate is a modified DNA oligonucleotide that emits a pink fluorescence when cleaved by DNase. For this assay, fluorescence was measured on a Tecan Ultra (excitation/emission 535/595 nm using medium gain). Solutions containing DNase activity produced a pink fluorescence, whereas solutions without DNase activity did not fluoresce. Thus, higher levels of DNase corresponded to an increase in the amount of light output. Negative controls consisted of nuclease-free water (80 µl) in place of sample. DNAase activity can be detected and quantified in a rate dependent manner using the 903 or FTA filter papers as solid supports. Figure 2a demonstrates that recovery of enzymes is possible on a FTA chemically treated filter paper, and Figure 2b demonstrates that recovery of enzymes is possible on a 903 untreated filter paper.

### Ribonuclease (RNase)

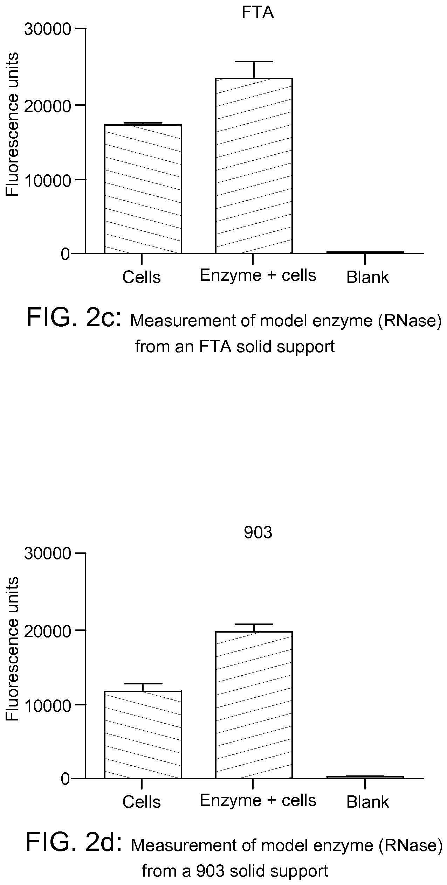

Detection of RNase was carried out as follows using a cleavable fluorescent-labelled RNase substrate. Each punch was ejected into separate wells of 96-well plates. Lyophilized RNase Alert Substrate was dissolved in TE buffer (1 ml) and dispensed (10 µl) into the test wells of the 96-well plate. 10X RNase Alert Buffer (10 µl) and nuclease-free water (80 µl) was added and the test solution (100 µl) incubated for 60 minutes at 37°C. The RNase Alert QC System Substrate is a modified RNA oligonucleotide that emits a green fluorescence when cleaved by RNase. For this assay, fluorescence was measured on a Tecan Ultra (excitation/emission 485/535 nm using medium gain). Solutions containing RNase produced a green fluorescence, whereas solutions without RNase activity did not fluoresce. Thus, higher levels of RNase corresponded to an increase in the amount of light output. Negative controls consisted of nuclease-free water (80 µl) in place of sample. Figures 2c and 2d demostare that RNAase activity can be detected and quantified in a rate dependent manner using the 903 or FTA papers.

### Example 3: Measurement of Lactic Dehydrogenase (LDH)

### Enzyme Assay for LDH

LDH is a cytosolic enzyme present in many different types of cells. When the plasma membrane is damaged, LDH is released into the bathing medium surrounding cells. The released LDH can be quantified by a coupled enzymatic reaction. First, LDH catalyzes the conversion of lactate to pyruvate via reduction of NAD+ to NADH. Second, diaphorase uses NADH to reduce a tetrazolium salt (INT) to a red formazan product. Therefore, the level of formazan formation is directly proportional to the amount of released LDH in the medium. The assay is performed by transferring a punch from with the addition of cells or LDH enzyme or into a microplate and adding the kit reagents (LDH Cytotoxicity Assay Kit; Thermo Scientific, Product Code 88953). After incubation at room temperature for 30 minutes, reactions are stopped and LDH activity is determined by spectrophotometric absorbance at 490nm.

### Immunoassay for LDH

An alternative more sensitive approach involves the use of immunoassay detection of LDH (LDHB human ELISA Kit (abcam product code 116693). This system involves the quantitative measurement of human LDHB protein in cell and tissue lysates using a punch in system as above. The assay employs an antibody specific for human LDHB coated on a 96-well plate. Samples are pipetted into the wells and LDHB present in the sample is bound to the wells by the immobilized antibody. The wells are washed and an anti-LDHB detector antibody is added. After washing away unbound detector antibody, HRP-conjugated label specific for the detector antibody is pipetted to the wells. The wells are again washed, a TMB substrate solution is added to the wells and colour develops in proportion to the amount of LDHB bound. The developing blue colour is measured at 600 nm. Optionally the reaction can be stopped by adding hydrochloric acid which changes the colour from blue to yellow and the intensity can be measured at 450 nm.

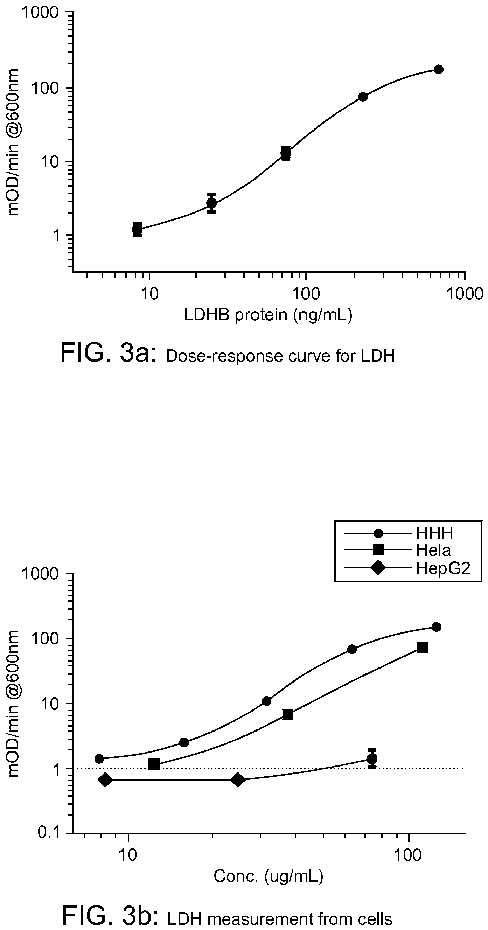

Figures 3a and 3b show LDH measurement results, and demonstrate that LDH can be detected and quantified for solid supports.

### Example 4: Direct Polymerase Chain Reaction (PCR) from blood preserved on Whatman FTA and 903 Cards

Thermo Scientific Phusion Blood Direct PCR Kit was demonstrated to support the amplification of DNA directly from blood samples stored on a range of solid supports including Whatman 903, FTA and FTA Elute cards (Chum and Andre 2013; Thermo Fisher Scientific). FTA and FTA elute cards are examples of chemically coated paper-based cards whilst 903 cards do not have any applied chemicals. In direct amplification workflows, no prior DNA extraction or purification steps are needed and excised portions of the cards are simply added to the PCR reaction mixture. Blood was chosen as the biological sample as this is considered to be the most challenging sample type from which to generate PCR amplicons. This is due to the presence of heam which is a potent PCR inhibitor (Akane et al 1994, J. Forensic Sci., 39, 362-372).

Sample preparation: Fresh blood or blood preserved with heparin (1.4 IU/ml), K2EDTA (1.8 mg/ml), or Na Citrate (109 mM) was applied to Whatman 903 Cards, FTA Elute Cards, or FTA Gene Cards and dried as per the manufacturer's instructions. For direct PCR, a 1 mm diameter disc was punched out of the sample and used in the following PCR reaction volumes: Whatman 903: 10-50 µl, FTA Elute Card: 25-50 µl and FTA Gene Card: 50 µl. When larger punches or smaller reaction volumes were used, punches were washed with 20 µl of H₂O at 50°C for 3 minutes. After removing the H₂O, PCR components were added directly to the rinsed punch. The parameters and reagents used are listed in tables II, III and IV, below.

**Table II- PCR reaction mixtures:**

| **Component** | **25 µL Reaction** | **50 µL Reaction** | **Final Conc.** |
|---|---|---|---|
| H₂O | Add to 25 µL | Add to 50 µL | |
| 2x Phusion Blood PCR Buffer | 12.5 µL | 25 µL | 1x |
| Primer F (Forward) | xµL | xµL | 0.5 µM |
| Primer R (Reverse] | xµL | xµL | 0.5 µM |
| Phusion Blood DNA Polymerase | 0.5µL | 1 µL | |
| 903/FTA Card | 1 mm punch | 1 mm punch | |

| **Optional Components for Reaction Optimization*** | | | |
|---|---|---|---|
| 50 mM MgCl₂ | 0.75 µL | 1.5 µL | |
| 50 mM EDTA | 0.6 - 1.25 µL | 1.25 - 2.5 µL | |
| DMSO | 1.25 µL | 2.5 µL | 5% |

**Table III- PCR thermo-cycling protcols- the two step protocol was used when primer Tm values were 69-72 degrees Celcius:**

| | **2-step Protocol** | | **3-step Protocol** | | |
|---|---|---|---|---|---|
| **Cycle Step** | **Temp.** | **Time** | **Temp.** | **Time** | **Cycles** |
| Lysis of cells | 98 °C | 5 minute | 98 °C | 5 minute | 1 |
| Denaturation | 98 °C | 1 s | 98 °C | 1 s | 35-40 |
| Annealing* | - | - | x °C | 5 s | |
| Extension** | 72 °C | 15-30 s/kb | 72 °C | 15-30 s/kb | |
| Final extension | 72 °C | 1 minute | 72 °C | 1 minute | 1 |
| | 4 °C | hold | 4° C | hold | |

**Table IV- Specific primers used to amplify the genes of interest:**

| **Gene of Interest** | **Amplicon** length (kb) | **Forward/Reverse Primer** | **Sequ ence listing** | **Annealing** Temperature (°C) |
|---|---|---|---|---|
| Cathepsin K gene | 0.5 | GAGAATCGCTTGAACCCGGG AGGTGTAGGT | 1 | 78.1 |
| | | CCTGCTGATGCCTGGCCTCT TTCTTCTTTG | 2 | 78.1 |
| Glutathione peroxidase 3 | 1.0 | CATCAGCCCGTCTAGGAACC CAGTCATCAG | 3 | 77.6 |
| | | CTCCTTCATCCCGCTACACCA CGCATACAC | 4 | 77.9 |
| Beta-globin gene | 3.8 | GCACTGGCTTAGGAGTTGGA CT | 5 | 65.9 |
| | | ACAGACACCCAGGCCTACTT G | 6 | 65.6 |
| Beta-globin gene | 7.5 | GCACTGGCTTAGGAG TTGGACTTCAAACC | 7 | 73.9 |
| | | CAACTGCTGAAAGAGATGCG GTGGG | 8 | 75.1 |
| SOX21 gene 5' region (Control Primers of Phusion blood direct PCR Kit) | 0.2 | AGCCCTTGGGGASTTGAATTG CTG | 9 | 73.5 |
| | | GCACTCCAGAGGACAGCRGT GTCAATA | 10 | 72.2175.3 {R = A/G) |

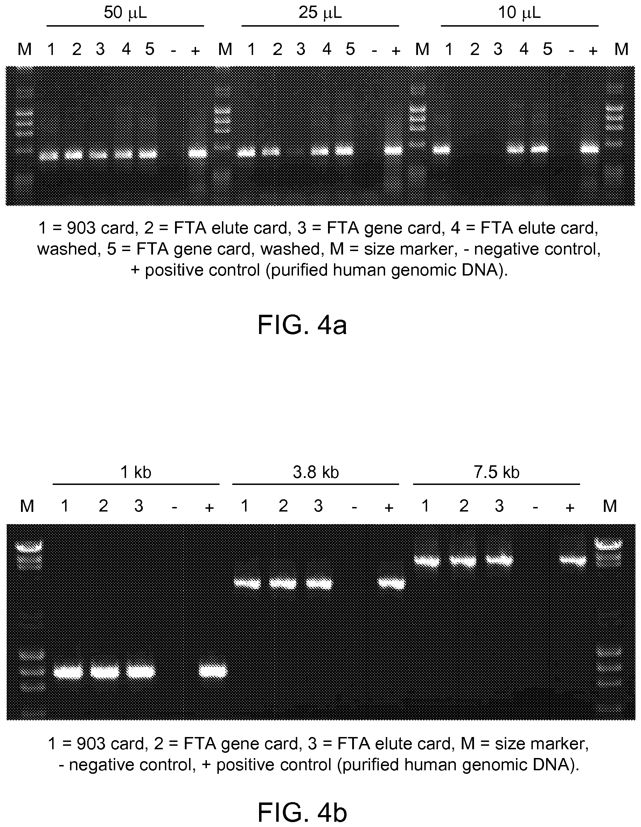

Figure 4a shows direct amplification of a 500 bp genomic DNA fragment from human blood treated with heparin and preserved on various cards. Reactions were performed from 1 mm punches either rinsed or placed directly into PCR reactions of 50, 25 or 10 µl in volume. A 2-step PCR protocol was used. A key is shown under the figure.

Figure 4b shows direct PCR of 1 kb, 3.8 kb and 7.5 kb gDNA amplicons from human blood treated with EDTA and preserved on various cards. Reactions were performed from 1 mm punches in 50 µl reactions (FTA Gene Card punches were rinsed as described in Materials and Methods for 7.5 kb fragment). A 2-step protocol was used for 1 kb and 7.5 kb fragments and a 3-step protocol for 3.8 kb amplicon. A key is provided under the figure.

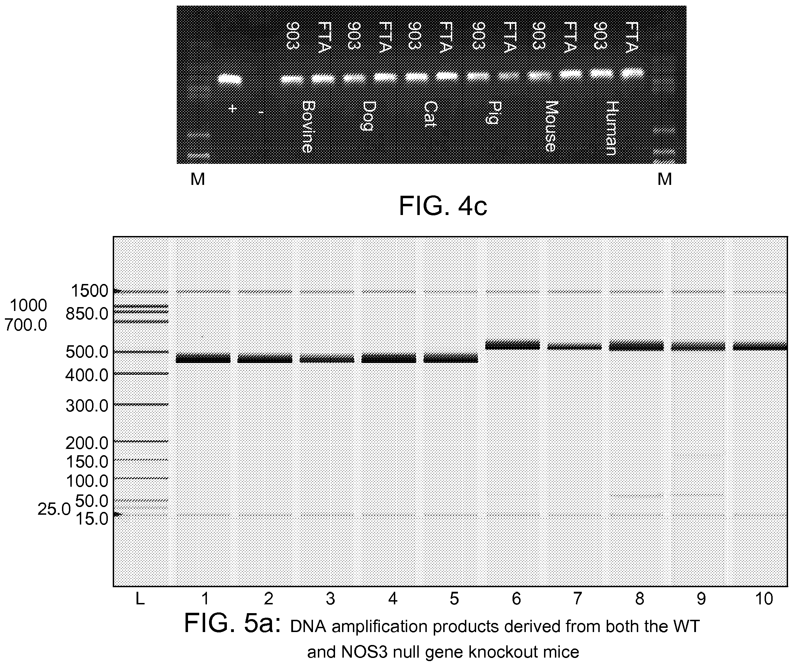

Figure 4c shows direct PCR performed on blood from several mammalian species treated with EDTA and preserved on 903 and FTA Gene Cards. Reactions were performed from 1 mm punches using the universal control primers included in the Phusion Blood Direct PCR Kit and 20 µl reaction volume (FTA Gene Cards were rinsed). M= Size Marker, - = Negative control, + = Positive control (purified human genomic DNA).

The PCR study confirmed that DNA can be directly amplified from biological samples stored on various filter cards. Samples derived from the 903 Cards showed almost no inhibition, and a 1 mm punch could be used with reaction volumes as low as 10 µl. Whatman FTA and FTA Elute filter paper (a variant of the Whatman FTA treated filter paper) exhibited varying degrees of inhibition. FTA elute inhibited direct PCR reactions slightly; a 1 mm disc in a 25-50 µl reaction worked well, but when placed in a 10 µl reaction, the PCR was totally inhibited. FTA Gene Cards showed the greatest level of inhibition. Without any pre-treatments, a 1 mm punch of FTA Gene Card worked well only in a 50 µl reaction volume. For smaller reaction volumes, a very simple washing protocol was enough to remove inhibitors from both FTA Elute and FTA Gene Cards. After washing the card punch for 3 minutes with H₂O, the sample was of sufficient purity for use in direct PCR reactions with Phusion Blood Direct PCR Kit at all reaction volumes tested.

Punches from 903 Cards and rinsed punches from FTA Elute and FTA Gene Cards (all 1 mm in diameter) were used in 50 µl reaction volumes with primers specific for 1 kb, 3.8 kb and 7.5 kb amplicons. In all cases, the PCR reaction generated the appropriately sized amplification product. The Phusion Blood Direct PCR Kit is compatible with blood from variety of species. A highly conserved 237 bp region upstream of the SOX21 gene (A. Woolfe, M. Goodson, PLoS Biol. 3, e7; 2004) was successfully amplified from blood of a number of vertebrate species dried onto 903 and FTA Gene Cards.

### Example 5: Genotyping using biological samples applied to solid support materials.

Many cancers are associated with genetic rearrangements. The Ewing sarcoma breakpoint region 1 (EWSR1) is translocated in many sarcomas. Recently, its rearrangement has been described in salivary gland hyalinizing clear cell carcinomas (Shah AA et al 2013 Am J Surg Pathol. 37:571-8 EWSR1 genetic rearrangements in salivary gland tumors: a specific and very common feature of hyalinizing clear cell carcinoma). The study described below illustrates the potential of solid support material and the idea described in this document to potentially screen for such genetic rearrangements within a complex mammalian genome.

### DNA Sample Collection, storage and detection

Murine tissues from c57BL/6 mice and NOS3 null mice (in a 129/B6 background) were applied to a range of different paper-based solid supports. The mice were euthanized and dissected to collect organs (blood, heart, brain, lung, liver, and kidney). The Organs were 'sandwiched' between two paper layers. Pressure was applied via a sterile pipette to imbed tissues in each of the cellulose matrices. For tissue homogenate, approximately 5 g of tissue was processed using a plastic dounce homogenizer in a 1.5 ml microfuge tube and then subsequently applied to the appropriate paper matrix. After application all the samples were allowed to air-dry for 2 hours prior to storage in a sealed pouch with desiccant. In some instances samples were stored up to 2 months before processing.

### DNA purification, genotyping, and quantitation

A Harris disposable micro punch (1.2 mm or 3 mm diameter) was used to excise the dried tissue samples from the paper cards respectively in the form of punched disks. The sample disk was excised from the centre of the dried sample and placed in a clean DNase free-1.5 ml micro-centrifuge tube. Null or gene knockout NOS3 mice were identified by PCR amplification of genomic DNA with endothelial Nitric Oxide Synthases (eNOS) exon 10-specific forward primer (see sequence listing 1), eNOS Neo-specific forward primer (sequence listing 2), and eNOS exon 12-specific reverse primer (see sequence listing 3). Target DNA's were amplified with an initial 10 min denaturation step followed by 36 cycles of 94°C for 35 sec, 650C for 1 min, and 72°C for 1 min; followed by a final extension at 72°C for 5 min. using a MJ Research thermo-cycler. The resultant PCR products were visualized with using an Experion capillary electrophoresis system. Mouse DNA quantification was achieved using the Primer Design genomic DNA quantification kit for mouse samples (gDNA-mo-q-DD) following manufacturer's instructions. Individual wild type (WT) and NOS null tissue samples were applied separately to different paper cards. In order to exemplify the ability to differentiate genotypic variants from DNA stored on the paper matrices, PCR amplification of a region was carried out on WT and transgenic (NOS3 null, gene knock-out) mice.

In Figure 5a PCR amplicons are shown, associated with the NOS locus using DNA as an amplification template isolated from tissues from the paper cards. Lanes 1-5 are DNA isolated from WT mouse tissue (Heart, Liver, Brain, Lung, and Kidney respectively). Lanes 6-10 are DNA amplified from NOS mouse tissues (Heart, Liver, Brain, Lung, and Kidney respectively).

**Table V:**

| DNA type | DNA Source | Solid support A | Solid support B | Solid support C | Solid support D |
|---|---|---|---|---|---|
| Wild Type Tissue DNA | Blood | + | ND | ND | ND |
| | Heart | ND | + | + | + |
| | Liver | ND | + | + | + |
| | Brain | ND | + | + | + |
| | Lung | ND | + | + | + |
| | Kidney | ND | + | + | + |
| Knock Out Tissue DNA | Blood | + | ND | ND | ND |
| | Heart | ND | + | + | + |
| | Liver | ND | + | + | + |
| | Brain | ND | + | + | + |
| | Lung | ND | + | + | + |
| | Kidney | ND | + | + | + |

In Table V above the successful amplification of DNA isolated from tissues stored on various solid supports A,B,C and D is recorded. DNA was isolated from a 1.2mm punch. '+' signifies the presence of amplicons. ND = not determined.

Figure 5a and Table V above show the DNA amplification products derived from both the WT and NOS3 null gene knock-out mice respectively. Results indicate that for both sample sources, the correctly sized DNA amplicons were produced from DNA isolated from all organ/tissue sources applied to the solid paper-support matrix. These data indicate that 1.2 mm Harris micro-punches can excise sufficient DNA from tissue stored on the solid paper supports to differentiate two genetic variants.

### Example 6: RNA collection, purification and quantitation

Tissue samples were applied to solid support paper cards as described sample punches were excised and the RNA isolated using the GE Healthcare illustra ® RNA spin kit as described below. RNA quantitation was performed on an ABI 7900 real time PCR system utilizing the commercially-available mRNA quantification kits.

Using a Harris 3 mm disposable micro punch, a punch was excised from the center of the dried sample spot and place in a clean RNase-free 1.5 ml micro-centrifuge tube. The illustra buffer RA1 (350 µl) was combined with 3.5 µl β-mercaptoethanol and the solution was added to the disc. The disc was homogenized using a 20 gauge needle. The resultant homogenate was transferred to the RNAspin Mini filter column for subsequent removal of residual material. The column was centrifuged for 1 min at 11,000 x g. and the RNAspin Mini Filter discarded. The homogenized lysate contains the RNA and this filtrate was transferred to a new RNase-free 1.5 ml micro-centrifuge tube.

Ethanol (70%; 350 µl) was added to the homogenized lysate and mixed by vortexing for 2 x 5 sec pulses. For each preparation, the lysate was pipette up-and-down 2-3 times, and applied to an RNA Mini-spin column placed in a 2 ml micro-centrifuge tube. The tubes were centrifuged for 30 sec at 8000 x g and the flow through discarded. The RNA spin column was transferred to a new collection tube.

The illustra MDB buffer (350 µl) was added and the tube centrifuged at 11 000 x g for 1 min. Once again the flow-through was discarded and the column returned to the collection tube. A DNase reaction mixture was prepared according to manufacturer's instructions and was added to the surface of the filter contained within the RNAspin column. This DNAse incubation was performed at room temperature for 15 min.

The wash buffer RA2 (200 µl) was applied to the RNA Mini-spin column and the column was centrifuged for 1 min at 11 000 x g. Once again the flow-through was discarded and the column returned to the collection tube.

Buffer RA3 600 µl was applied to the RNA Mini-spin column and the column centrifuge for 1 min at 11 000 x g the flow-through was discarded and the column returned to the collection tube. An addition column wash with buffer RA3 (250 µl) was also performed. In order to dry the membrane completely, the column was centrifuged for 2 min at 11 000 x g and the column finally placed into a nuclease-free 1.5 ml micro-centrifuge tube.

RNase-free water (40 µl) was applied to the column and the column centrifuged at 11 000 x g for 1 min. The purified RNA was either used immediately in downstream applications or stored at -80°C until used.

To determine the integrity of RNA from multiple tissues after prolonged storage, real-time reverse transcription polymerase chain reaction (RT-PCR) was carried out on RNA isolated from mouse tissue samples stored on the paper cards. These were stored in the presence of a desiccant for 2 months. mRNA quantification was accomplished according to manufacturer's instructions using either i) the ABI Taqman rodent GAPDH control kit (part # 4308313), ii) the Invitrogen real-time LUX mRNA primer sets for murine HPRT, GAPDH, and Beta-Actin genes (cat. 105M-02, 100M-02, and 101M-02 respectively) or iii) tissue specific gene primer sets from Applied Bio-systems.

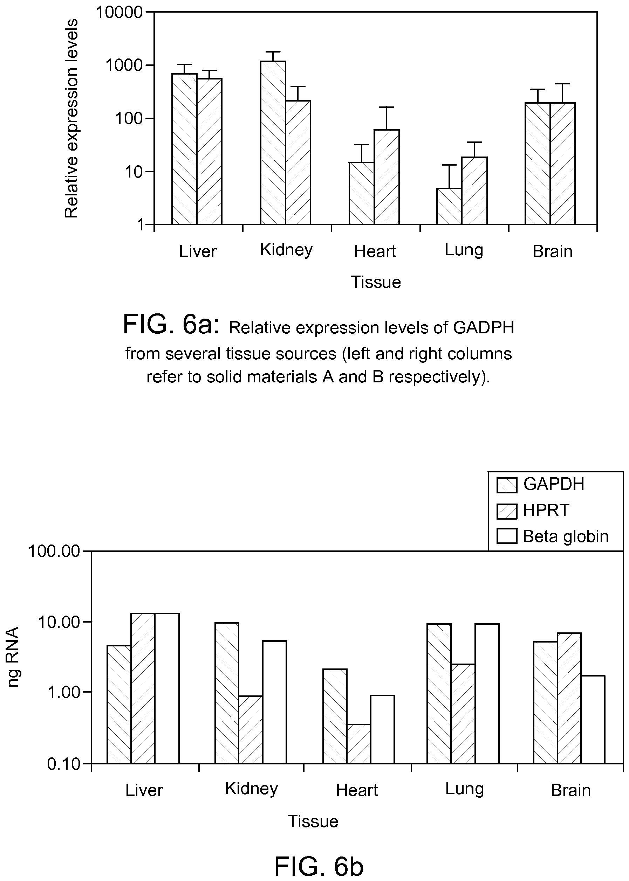

Figure 6a shows the relative expression levels of GADPH from several tissue sources using the ABI Taqman rodent GAPDH control kit. RNA levels derived from samples applied to solid support cards (Support material A and B) were determined by comparison to known values generated from a quantification titration curve from mouse RNA standard samples. Comparable GAPDH RNA levels were detected from RNA isolated from both paper types.

Absolute quantitation of murine mRNA encoding HPRT, GAPDH and Beta-Actin was carried out with the appropriate Invitrogen real-time LUX primer sets. RNA levels derived from samples applied to paper support material A were determined by comparison to known values generated from a quantification titration curve from mouse RNA standard samples. Murine RNA recovery data associated with the isolation of RNA is described in Figure 6b and demonstrate that is material is able to support the storage and stabilization of RNA from numerous tissue types. Similar observations were apparent for RNA stored on support material B.

In summary the examples above demonstrate that it is possible to recover, from solid supports of differing constructions, a wide range of analytes which have the same or similar biological structure to the OSCC biomarkers of interest given in Table I.

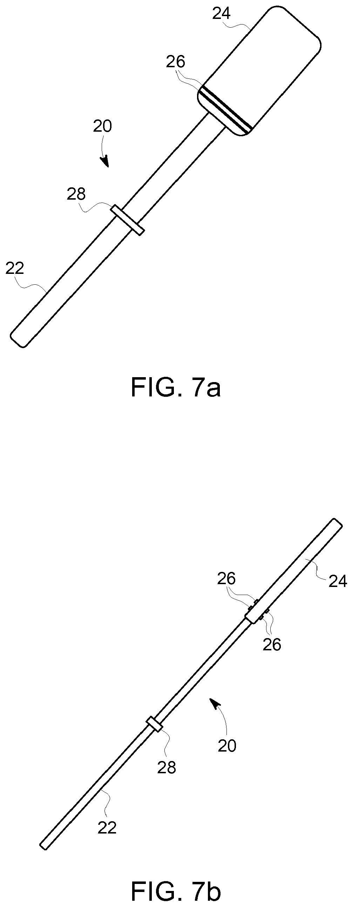

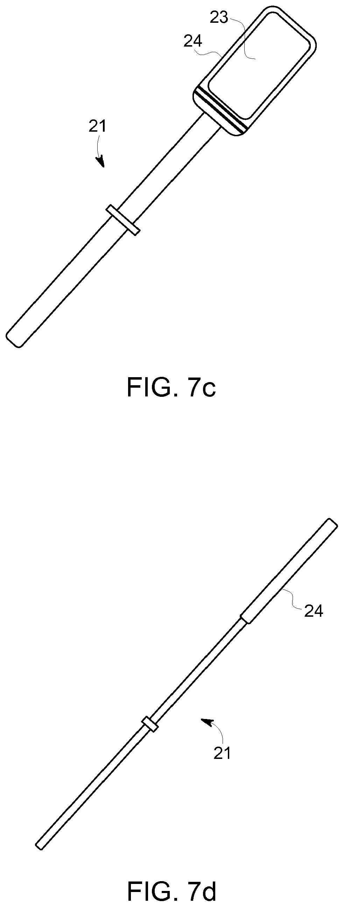

A suitable swab device which will include a solid support collector is described in more detail below with reference to Figures 7 to 12.

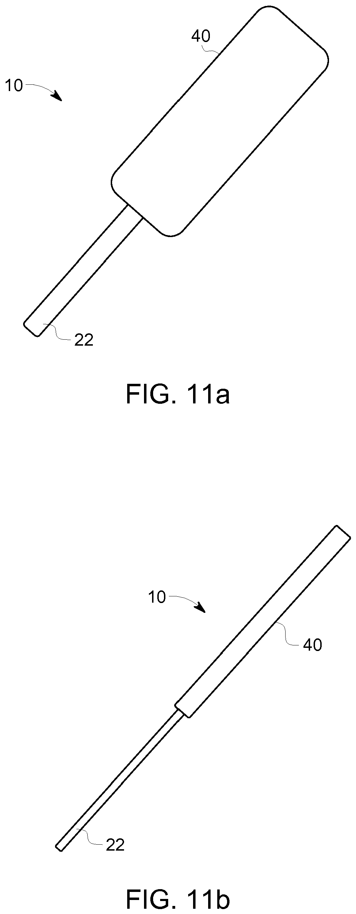

Figures 7a and 7b show plan and side views respectively of a spine member 20 for a swab 10 (shown assembled in Figure 11a). The spine 20 includes a handle portion 22 and an end paddle 24, which is wider than the handle 22 in plan, but about the same width as the handle when viewed from the side. The spine can be injection molded in one piece. Onto the paddle, adjacent the handle, on opposing sides is deposited pressure sensitive adhesive 26 of attaching a solid support for collecting biological material samples. In this case two strips 26 are employed. The handle 22, approximately half way along its length, has a protruding formation 28, for attaching a cover 40 shown in Figure 11a.

Figures 7c and 7d show plan and side views respectively of an alternative spine member 21, which has the same configuration as the spine 20, expect that the spine 21 has a window 23 in the paddle 24.

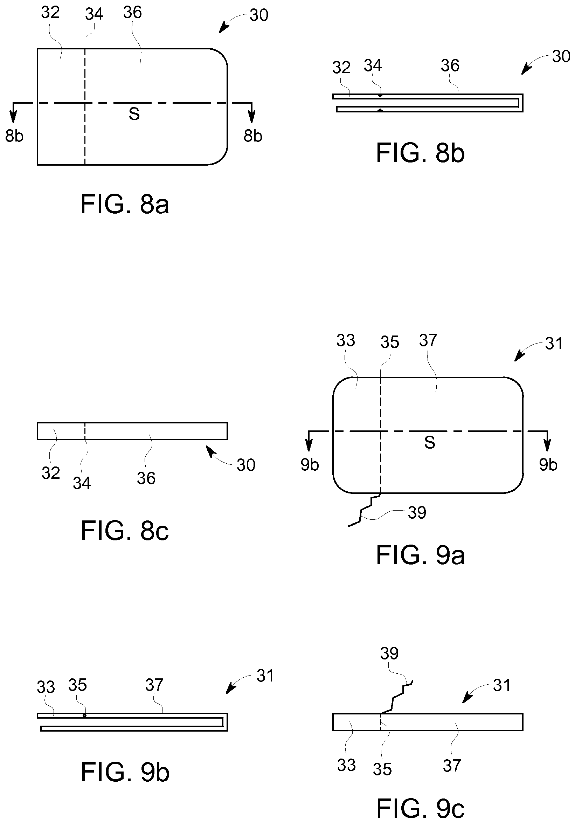

Figure 8a shows a plan view of a solid support 30 , of the type described above, suitable for collecting saliva samples, and suitable for affixing to the paddle 24 by means of the adhesive strip(s) shown in Figures 7a and 7b. Figure 8b shows the paper in cross section along line 8b-8b shown in Figure 8a, and Figure 8c shows a side view of the solid support. In this case the solid support is a cellulose paper sheet, for example of the type sold under the trade name Whatman 903, which has been folded and sealed at its edges. The paper has a line of weakness 34 and an end region 32 which is intended to be fixed to the paddle by means of adhesive 26. The end region 36 is used to collect a sample S.

Figure 9a shows a plan view of a variant solid support 31, of the type described above, suitable for collecting saliva samples, and suitable for affixing to the paddle 24 by means of the adhesive strip(s) shown in Figures 7a to 7d. Figure 9b shows the paper in cross section along line 9b-9b shown in Figure 9a, and Figure 9c shows a side view of the solid support. The solid support is the same folded cellulose paper sheet. The paper has a perforated line of weakness 35 and an end region 33 which is intended to be fixed to the paddle by means of adhesive 26. A sample collection region 37 lies in the same area as the region 36. The variant includes a cord 39 which lies under the paper when folded, but protrudes beyond one edge, so that it can be grasped in use and pulled to tear the paper and release the sample region 39 in use.

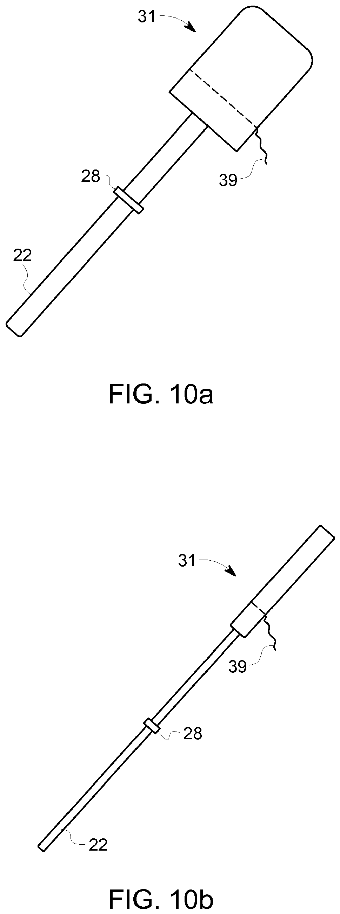

Figures 10a and 10b show plan and side views of the spine 20 and solid support 31 assembled, affixed together by means of adhesive 26 (not visible). An assembly with the solid support 30, although illustrated, would be the same.

Figures 11a and 11b show plan and side views of the assembly shown in Figures 10a and 10b, covered with a removable cover 40, which is attached to the handle 22 at the protrusion 28 shown in Figure 7a.

In use the swab 10 is held and manipulated by means of the handle 22. The cover can be removed to reveal the solid support 30 or 31 ready for use. A saliva sample can be taken (which could be a self-sample), and the cover replaced by the sample taker, for sending to a laboratory for further processing. At the laboratory, the solid support can be removed from the remaining swab parts, and then a portion for the paper can be removed for testing, for example by using a punch. If the spine 21 is used, then the portion solid support can be removed without removing the solid support from the spine because a punch or similar can be made through the window 23.

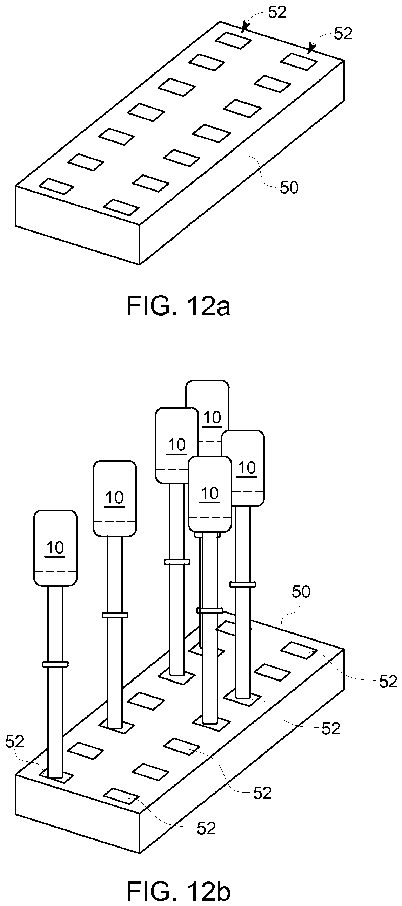

Where multiple samples are taken, then a drying rack 50 can be used as illustrated in Figures 12a and 12b, wherein multiple swabs 10 are place handle down in wells 52 in the rack, with their covers 40 removed.

Each solid support 30/31 is tested ex vivo for the presence of two or more of the biomarkers of interest according to any one of the techniques described above, indicative of oral cancerous activity.

For example a plain 903 paper solid support 30/31 is described, but other solid supports could be used. For example filter paper treated with preserving chemicals could be used, as sold under the brand name Whatman FTA or FTA Elute, each of which are pretreated. FTA is pretreated with a stabilising reagent mix comprising: a weak base, and a chelating agent, uric acid or a urate salt, and an anionic surfactant, such as odium dodecyl sulphate (SDS) and/or sodium lauryl sarcosinate (SLS). FTA Elute is pretreated with stabilising reagent mix comprising a chaotropic salt in the form of guanidinium thiocyanate. Where FTA or FTA Elute are used, the sapling technique may involve taking a saliva sample and then transferring it to the solid support to avoid direct contact between the oral cavity and the treated solid support. Where stabilising reagent are used, it has been found that the addition of cyclodextrin in the detection step acts as a sequesterant, and thereby improves the efficiency of direct PCR, qPCR and other nucleic acid amplification techniques.

A solid support formed from cellulose paper is preferred.

## Claims

1. An *ex vivo* method for detecting plural biomarkers within biological material present in a human oral cavity when oral cancerous activity is present in said cavity, the method comprising the steps of:
i) providing a solid cellulose based paper support for accepting a sample of biological material from the oral cavity;
ii) depositing a sample of the biological material on the solid support,
iii) performing *ex vivo* plural assays and detect the presence of the p53 gene codon 63 in the biological material deposited on the solid support, wherein the assay includes the step of removing a portion of the solid support,using a punch; and further including the step of quantifying at least one of said plural biomarkers.

2. A method as defined in claim 1, wherein said biomarkers are a plural selection from those candidates listed in Table I herein.

3. A method as claimed in claim 1 or 2, wherein said plural selection are in the form of one or more nucleic acids, and/or one or more proteins, and/or one or more enzymes or other analytes indicative of said oral cancerous activity.

4. A method as claimed in any one of claims 1 to 3, wherein said one or more assays, includes one or more assays selected from the group consisting of: gene expression or protein expression profiling using RT-PCR, polymerase chain reaction (PCR), quantitative PCR (qPCR), isothermal amplification, immunological-PCR, microarray assays, enzyme linked immunosorbent assay (ELISA), immunological techniques, gel electrophoresis (2DE), capillary electrophoresis (TOF MS), high performance liquid chromatography (HPLC), mass spectrometry (MS), flame photometry, atomic absorption spectrophotometry, and visible spectrophotometry.

5. A method as claimed in any one of claims 1 to 4, wherein the solid support is optionally chemically treated with a stabilising reagent or reagent mix.

6. A method as claimed in claim 5, wherein said reagent or reagent mix comprises: a weak base, and a chelating agent, optionally, uric acid or a urate salt, and optionally an anionic surfactant, or comprises: a chaotropic substance such as a chaotropic salt, for example guanidinium thiocyanate.

7. A method as claimed in claim 5 or 6, where the solid support is chemically treated, the step ii) of claim 1 includes taking a biological material sample from an oral cavity, and transferring said sample onto said chemically treated solid support without said chemically treated solid support contacting the oral cavity.

8. A method as claimed in claim 7 wherein, step iii) of claim 1 is performed in the presence of cyclodextrin.

9. A method as claimed in one of claims 1 to 8, further including the step of providing a swab for mounting the solid support, said swab comprising: a spine, including a handle portion and a paddle portion at one end of the handle for holding a solid support.

## Patentansprüche

1. Ex-vivo-Verfahren zum Nachweis mehrerer Biomarker in biologischem Material, das in einer menschlichen Mundhöhle vorhanden ist, wenn eine orale Krebsaktivität in der Höhle vorhanden ist, wobei das Verfahren die folgenden Schritte umfasst:
i) Bereitstellen eines festen Papierträgers auf Zellulosebasis zur Aufnahme einer Probe biologischen Materials aus der Mundhöhle;
ii) Aufbringen einer Probe des biologischen Materials auf dem festen Träger,
iii) Durchführen von mehreren Ex-vivo-Assays und Nachweis des Vorhandenseins des p53-Gen-Codons 63 in dem auf dem festen Träger aufgebrachten biologischen Material, wobei der Assay den Schritt des Entfernens eines Abschnitts des festen Trägers unter Verwendung eines Stanzwerkzeugs beinhaltet; und weiter einschließend den Schritt der Quantifizierung mindestens eines der mehreren Biomarker.

2. Verfahren nach Anspruch 1, wobei die Biomarker eine Mehrfachauswahl aus den in Tabelle I aufgeführten Kandidaten sind.

3. Verfahren nach Anspruch 1 oder 2, wobei die Mehrfachauswahl in Form von einer oder mehreren Nukleinsäuren und/oder einem oder mehreren Proteinen und/oder einem oder mehreren Enzymen oder anderen Analyten erfolgt, die die orale Krebsaktivität anzeigen.

4. Verfahren nach einem der Ansprüche 1 bis 3, wobei der eine oder die mehreren Assays einen oder mehrere Assays beinhalten, ausgewählt aus der Gruppe bestehend aus: Genexpressions- oder Proteinexpressionsprofiling unter Verwendung von RT-PCR, Polymerase-Kettenreaktion (PCR), quantitativer PCR (qPCR), isothermer Amplifikation, immunologischer PCR, Microarray-Assays, enzymverknüpftem Immunosorbent-Assay (ELISA), immunologischen Techniken, Gelelektrophorese (2DE), Kapillarelektrophorese (TOF MS), Hochleistungsflüssigkeitschromatographie (HPLC), Massenspektrometrie (MS), Flammenphotometrie, Atomabsorptionsspektroskopie und Spektrophotometrie im sichtbaren Bereich.

5. Verfahren nach einem der Ansprüche 1 bis 4, wobei der feste Träger optional chemisch mit einem stabilisierenden Reagenz oder Reagenziengemisch behandelt wird.

6. Verfahren nach Anspruch 5, wobei das Reagenz oder die Reagenzmischung umfasst: eine schwache Base und ein Chelatisierungsmittel, optional Harnsäure oder ein Harnsalz, und optional ein anionisches Tensid, oder wobei es umfasst: eine chaotrope Substanz, wie ein chaotropes Salz, zum Beispiel Guanidiniumthiocyanat.

7. Verfahren nach Anspruch 5 oder 6, bei dem der feste Träger chemisch behandelt wird, wobei der Schritt ii) von Anspruch 1 das Entnehmen einer Probe aus einer Mundhöhle und das Übertragen der Probe auf den chemisch behandelten festen Träger einschließt, ohne dass der chemisch behandelte feste Träger die Mundhöhle berührt.

8. Verfahren nach Anspruch 7, wobei Schritt iii) von Anspruch 1 in Gegenwart von Cyclodextrin durchgeführt wird.

9. Verfahren nach einem der Ansprüche 1 bis 8, weiter umfassend den Schritt des Bereitstellens eines Abstrichtupfers zum Befestigen des festen Trägers, wobei der Abstrichtupfer umfasst: ein Stäbchen einschließend einen Griffabschnitt und einen Paddelabschnitt an einem Ende des Griffs zum Halten eines festen Trägers.

## Revendications

1. Procédé *ex vivo* permettant de détecter plusieurs biomarqueurs dans une matière biologique présente dans une cavité buccale humaine lorsqu'une activité cancéreuse buccale est présente dans ladite cavité, le procédé comprenant les étapes de :
i) fourniture d'un support papier solide à base de cellulose destiné à recevoir un échantillon de matière biologique provenant de la cavité buccale ;
ii) dépôt d'un échantillon de la matière biologique sur le support solide,
iii) réalisation de plusieurs dosages *ex vivo* et détection de la présence du codon 63 du gène p53 dans la matière biologique déposée sur le support solide, dans lequel le dosage inclut l'étape d'extraction d'une partie du support solide, à l'aide d'un poinçon ; et incluant en outre l'étape de quantification d'au moins l'un desdits plusieurs biomarqueurs.

2. Procédé selon la revendication 1, dans lequel lesdits biomarqueurs sont une sélection plurielle parmi les candidats recensés au tableau 1 de la présente demande.

3. Procédé selon les revendications 1 ou 2, dans lequel ladite sélection plurielle se présente sous la forme d'un ou plusieurs acides nucléiques et/ou d'une ou plusieurs protéines et/ou d'une ou plusieurs enzymes ou d'autres analytes indiquant ladite activité cancéreuse buccale.

4. Procédé selon l'une quelconque des revendications 1 à 3, dans lequel lesdits un ou plusieurs dosages incluent un ou plusieurs dosages sélectionnés dans le groupe comprenant : le profilage de l'expression génétique ou de l'expression protéique par RT-PCR, la réaction en chaîne de la polymérase, la PCR quantitative (qPCR), l'amplification isotherme, la PCR immunologique, les dosages en microréseaux, le dosage immunoenzymatique (ELISA), les techniques immunologiques, l'électrophorèse sur gel (2DE), l'électrophorèse capillaire (TOF MS), la chromatographie en phase liquide à haute performance (HPLC), la spectrométrie de masse (MS), la photométrie à flamme, la spectrophotométrie d'absorption atomique et la spectrophotométrie visible.

5. Procédé selon l'une quelconque des revendications 1 à 4, dans lequel le support solide est éventuellement traité chimiquement avec un réactif ou un mélange de réactifs de stabilisation.

6. Procédé selon la revendication 5, dans lequel ledit réactif ou mélange de réactifs comprend : une base faible et un agent de chélation, éventuellement de l'acide urique ou un sel d'urate, et éventuellement un tensioactif anionique, ou comprend : une substance chaotrope tel qu'un sel chaotrope, par exemple du thiocyanate de guanidine.

7. Procédé selon les revendications 5 ou 6, où le support solide est traité chimiquement, l'étape ii) de la revendication 1 inclut le prélèvement d'un échantillon de matière biologique à partir d'une cavité buccale et le transfert dudit échantillon sur ledit support solide traité chimiquement sans que ledit support solide traité chimiquement soit au contact de la cavité buccale.

8. Procédé selon la revendication 7, dans lequel l'étape iii) de la revendication 1 est effectuée en présence de cyclodextrine.

9. Procédé selon l'une des revendications 1 à 8, comprenant en outre l'étape de fourniture d'un écouvillon pour fixer le support solide, ledit écouvillon comprenant : une tige incluant une partie manche et une partie spatule à une extrémité du manche pour tenir un support solide.
